# FASCICULE DE BREVET EUROPEEN

(11) **EP 1 059 904 B1**
(45) Date de publication et mention de la délivrance du brevet: **21.07.2004**
(21) Numéro de dépôt: 99937864.9
(22) Date de dépôt: 05.03.1999
(51) Int. Cl.: A61F 7/00, F16C 7/02, F25D 3/10

(54) **APPAREIL AUTONOME ET PORTABLE DE CRYOGENIE UTILISANT L'ANHYDRIDE CARBONIQUE EN PHASE LIQUIDE/SOLIDE**
AUTONOME UND TRAGBARE KRYO-VORRICHTUNG MIT VERWENDUNG VON KOHLENDIOXID IN FESTER / FLÜSSIGER PHASE
AUTONOMOUS AND PORTABLE CRYOGENIC APPARATUS USING CARBON DIOXIDE IN LIQUID/SOLID PHASE

(30) Priorité: 06.03.1998 FR 9802757
(43) Date de publication de la demande: 20.12.2000
(73) Titulaire: CRYONIC MEDICAL, 39110 Salins Les Bains (FR); VALLOUREC COMPOSANTS AUTOMOBILES VITRY, 51300 Vitry le Francois (FR)
(72) Inventeur: CLUZEAU, Christian, F-39110 Salins les Bains (FR); DESBROSSE, Jacky, F-51300 Huiron (FR)
(74) Mandataire: Bruder, Michel
(86) Numéro de dépôt international: PCT/FR1999/000508
(87) Numéro de publication internationale: WO 1999/044551

(56) Documents cités:
- EP-A- 0 633 008

## Description

L'invention concerne un appareil autonome et portable de cryogénie particulièrement bien adapté à la cryothérapie se définissant comme la thérapie par le froid très efficace dans le traitement de diverses affections que l'on peut regrouper en quatre grandes catégories :
- la diminution de la douleur : on sait en effet que la transmission des informations par les capteurs sensitifs du corps est considérablement ralentie par le froid, dans la mesure où le flux électrique généré par les nocicepteurs dans les fibres nerveuses se trouve lui-même considérablement ralenti et donc la douleur est atténuée,
- traitement des inflammations : on sait qu'un traumatisme ou un rhumatisme produit des enzymes inflammatoires et que cette production est fortement diminuée par un traitement cryothérapique,
- stimulation de la circulation : on sait aussi que le froid entraîne un réflexe vasomoteur et une intensification de l'action de drainage,
- détente musculaire : on sait enfin que le froid permet d'obtenir une réponse réflexe en profondeur en intervenant sur l'abaissement du tonus musculaire ; à cet égard, il est rappelé que le froid est beaucoup plus efficace que la chaleur à la condition toutefois de traiter avec un froid intense qui doit en outre provoquer une chute de température très rapide. Jusqu'ici, les appareils de cryothérapie utilisaient comme sources de froid soit l'air réfrigéré, soit plus généralement l'azote liquide dont la mise en oeuvre reste soumise à de nombreuses contraintes limitant le plus souvent la mobilité de l'appareil de traitement. Plus particulièrement dans le domaine du sport où la cryothérapie trouve une de ces applications majeures, on utilise exclusivement ou presque, des petites bombes de gaz sous basse pression, dont la détente aux abords d'un traumatisme local produit un froid n'excédant pas - 18°C avec une chute de température relativement lente.
   C'est pourquoi il a déjà été proposé dans le brevet européen EP-0.633.008 au nom du demandeur d'utiliser comme source de froid pour la cryothérapie, de l'anhydride carbonique (CO₂) comprimé dont une caractéristique intrinsèque est de procurer une température de détente de
- 78°C à la pression atmosphérique. Selon les enseignements de ce brevet, l'utilisation d'anhydride carbonique sous forme d'un mélange liquide/vapeur permet de maintenir une pression constante dans le récipient pendant sa vidange ; cette pression qui n'est autre que la pression de vapeur saturante dépend directement de la température du récipient. Ainsi, en se détendant jusqu'à la pression atmosphérique, l'anhydride carbonique se trouve sous deux phases : solide et gazeuse. C'est la phase solide, également appelée carboglace, qui permet de soutirer le maximum de chaleur au niveau de la peau du patient à traiter. En effet, au contact de celle-ci, la carboglace se sublime, évacuant ainsi une grande quantité de chaleur. Il est donc essentiel de soutirer du récipient la phase liquide de l'anhydride carbonique pour obtenir à la fois un froid intense et une chute de température extrêmement rapide.

Pour parvenir à ce résultat, l'appareillage selon le brevet antérieur prévoit à l'intérieur d'une bouteille d'anhydride carbonique liquéfiée sous pression, d'installer à partir de la tête de la bouteille, un tube plongeur s'étendant sur toute la longueur de la bouteille. Ainsi, soutire-t-on la phase liquide du CO₂ qui est ensuite projetée dans une canalisation souple grâce à un système de détente et d'éjection muni d'une manette de commande et d'un diffuseur permettant d'ajuster la forme, la dimension et la température du jet de CO₂ sur la zone à traiter.

Il s'agit d'un appareillage lourd, essentiellement destiné aux professionnels tels que kinésithérapeutes, totalement inadapté à un environnement autre que les services conventionnels de traumatologie, comme par exemple, les terrains de sport, les champs de courses ou encore les haras.

Pour surmonter ces inconvénients, il est donc proposé conformément à l'invention, un appareillage autonome, léger et portable, susceptible, de produire un froid intense procurant une chute de température très rapide, et ce, dans des conditions de sécurité thermique maximales. En effet, on comprend facilement que la projection de carboglace sur la peau d'un patient doit être sévèrement contrôlée, dans la mesure où la température superficielle au niveau de la peau ne doit jamais descendre en dessous de 0°C pour éviter d'endommager les tissus organiques. Pour assurer à l'appareil conforme à l'invention, une totale autonomie, il est donc impérieux que la mesure de la température de la zone ainsi traitée se fasse sans contact avec le patient mais en relation directe avec l'appareil portable afin d'éviter des manipulations qui ralentiraient considérablement l'intervention. A cet égard, il est proposé conformément à l'invention, un appareil portable de cryogénie utilisant à titre principal la température de détente (de l'ordre de -78°C à la pression atmosphérique) de l'anhydride carbonique (CO₂ ) ou équivalent en phase liquide/solide comportant un réservoir de CO₂ liquéfié sous pression dont la tête est reliée à un système de détente et d'éjection du CO₂ liquide/solide, et comportant des organes de commande, de détente et de contrôle de température de la zone d'utilisation, qui est remarquable en ce que la tête du réservoir de CO₂ est agencée sur l'appareil de telle manière que pendant toute la durée d'utilisation, seule la partie liquide du CO₂ à l'intérieur du réservoir vienne au contact de ladite tête pour y être soutirée et propulsée vers la zone d'utilisation par l'intermédiaire d'un système de détente et d'éjection.

Dans ce sens, l'appareil selon l'invention se caractérise en ce que le réservoir ayant avantageusement la forme générale d'une classique cartouche de gaz comprimé munie à son extrémité d'une tête de soutirage spéciale comprenant des moyens de solidarisation et de désolidarisation avec l'appareil et des organes de mise en communication avec son système de détente et d'éjection, est situé au-dessus de l'appareil, lorsqu'il est en position d'intervention, la cartouche étant inclinée dans le plan vertical et la tête de soutirage étant tournée vers le bas en direction de l'appareil auquel elle est solidarisée.

Selon une autre caractéristique tout à fait essentielle de l'appareil conforme à l'invention, il est prévu de l'équiper d'un système de mesure de température afin de prévenir l'opérateur lorsque la température de surface de la zone d'utilisation a atteint un seuil critique par exemple en cryothérapie lorsque la peau se rapproche de 0°C. Parmi les techniques existantes permettant de mesurer une température, les plus intéressantes en cryothérapie peuvent être regroupées en trois catégories : les sondes résistives, les timbres thermo-sensibles autocollants ou encore les pyromètres à distance et en particulier les pyromètres à infrarouge ; cette dernière catégorie, fonctionnant selon le principe que tout corps émet un rayonnement proportionnel à sa température à la puissance 4, correspond bien au caractère autonome et portable de l'appareil selon l'invention ; en outre, les pyromètres à infrarouge sont tout à fait à même d'effectuer des mesures extrêmement rapides, en rapport précis avec la zone de traitement, sous réserve toutefois de prévoir une alimentation électrique et de bien adapter la distance focale du pyromètre ; on sait en effet que si la distance de traitement est trop courte (par exemple de l'ordre de 3 à 4 centimètres) le gradient thermique de la peau soumise à un traitement de cryothérapie est tellement important que la température de la zone traitée et la température de la zone scrutée par le pyromètre sont alors très éloignées, ce qui ne permet pas la mise en oeuvre d'alarme sonore ou visible, avec la conséquence d'une mise en danger du sujet traité. Ainsi et selon une autre caractéristique importante de l'invention, il est prévu de disposer entre l'appareil et la zone d'utilisation, un guide d'approche permettant à coup sûr de placer le pyromètre à la distance focale voulue ; selon une exécution préférée, le guide d'approche sera constitué d'une simple tige coulissante donnant la distance entre le pyromètre et la zone à traiter ; cette tige peut en outre contribuer à la sécurité de l'appareil ; par exemple, la mise sous tension de l'appareil ne pourra être obtenue que lorsque cette même tige sera mise en position externe de mesure, comme expliqué plus loin ; de cette façon on limite les utilisations intempestives par les enfants, notamment.

Selon une autre caractéristique des pyromètres à infrarouge, il est tout à fait possible pour l'Homme du Métier de calculer la distance focale du pyromètre de telle manière que le spot de mesure sur la partie à traiter se situe toujours en dehors de la zone de projection de la carboglace, de telle manière que la mesure se fasse bien sur une zone traitée et ne prenne pas la température de la carboglace, ce qui serait le cas si le spot de mesure se superposait avec la zone de sublimation du CO₂ .

D'autres caractéristiques et avantages ressortiront mieux encore de la description d'un appareil autonome et portable de cryogénie, donnée à titre d'exemple préféré mais en aucun cas limitatif, en référence aux dessins sur lesquels :
- la figure 1 représente l'appareil en élévation et selon une coupe verticale montrant schématiquement la forme générale de l'appareil portable ainsi que la disposition relative de ses principaux composants,
- la figure 2 représente la vue de face correspondant à la figure précédente,
- la figure 3 représente la vue de dessus correspondant à la figure 1, montrant avec arrachement partiel, la disposition du pyromètre et son électronique de commande,
- la figure 4 est une représentation partielle à plus grande échelle de la tête de soutirage de la cartouche de CO₂ montée sur son support juste avant la percussion de l'opercule de fermeture de la cartouche,
- la figure 5 est la même que la figure précédente sauf que la cartouche est maintenant totalement engagée sur son support après percussion de son opercule.

Conformément aux figures, l'appareil de cryogénie 1 qui va être maintenant décrit est particulièrement destiné à la cryothérapie de l'homme ou de l'animal.

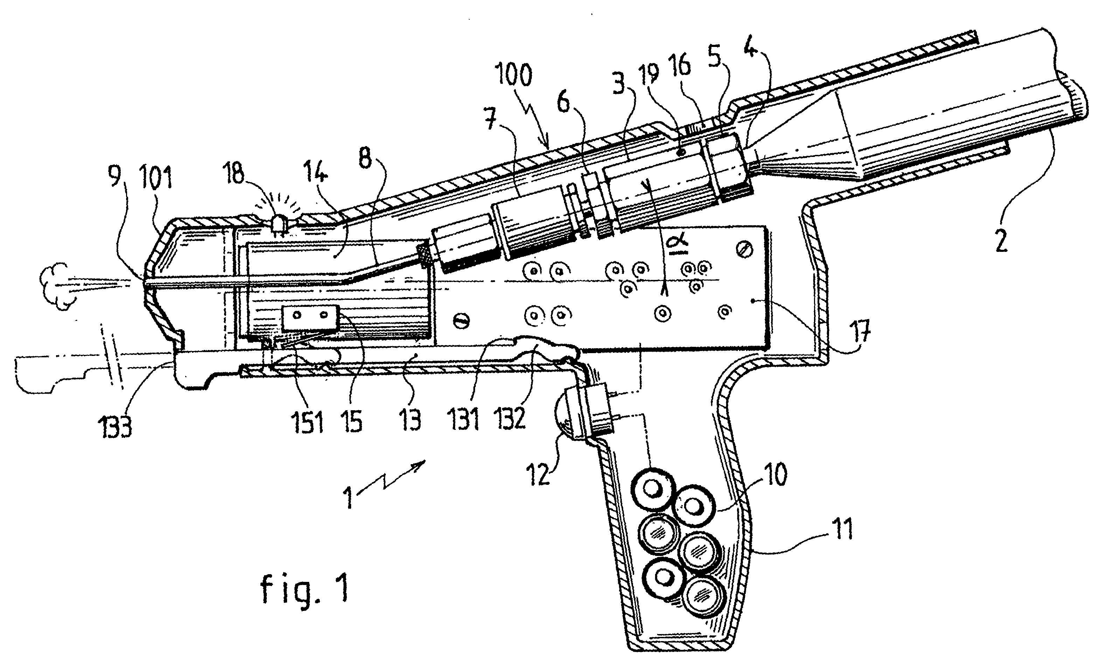

Selon la figure 1, l'appareil 1 a la forme générale d'un pistolet réalisé dans une coque plastique agencée intérieurement pour contenir les organes fonctionnels de l'appareil 1 ; celui-ci est alimenté par une cartouche de CO₂ en phase liquide, introduite à l'arrière selon une inclinaison verticale qui sera précisée plus loin. Le corps 100 du pistolet recevant la chaîne de détente cryogénique du pistolet comprend à l'arrière un support 3 accueillant d'un côté la tête de soutirage 4 de la cartouche 2 par l'intermédiaire d'une pièce de liaison 5, et de l'autre côté, un filtre 6 qui sera précisé plus loin, une électrovanne en ligne 7 mettant en communication l'anhydride carbonique CO₂ avec un tube de détente et d'éjection 8 coudé pour déboucher à l'extrémité avant 9 du canon 101 sensiblement horizontal et projeter le CO₂ en phase liquide/solide vers la zone d'utilisation (non représenté sur les figures).

L'électrovanne 7 fonctionnant en mode "tout ou rien" est d'un type tout à fait connu par exemple disponible au catalogue de la société suisse *"Fluid Automation Systems".* Elle est alimentée sous tension continue de 12 volts par un jeu de batteries 10 classiquement aménagées à l'intérieur de la crosse 11 servant de poignée pour le pistolet 1, suivant un empilage d'éléments donnant une sécurité de fonctionnement électrique maximale. Naturellement, pour actionner l'électrovanne 7 et par conséquent distribuer le CO₂ sous forme liquide/solide au point de sortie 9 du pistolet, il est nécessaire d'actionner un interrupteur 12 disposé à l'avant de la crosse 11, à la manière d'une gâchette classique par l'index de l'utilisateur tenant le pistolet 1.

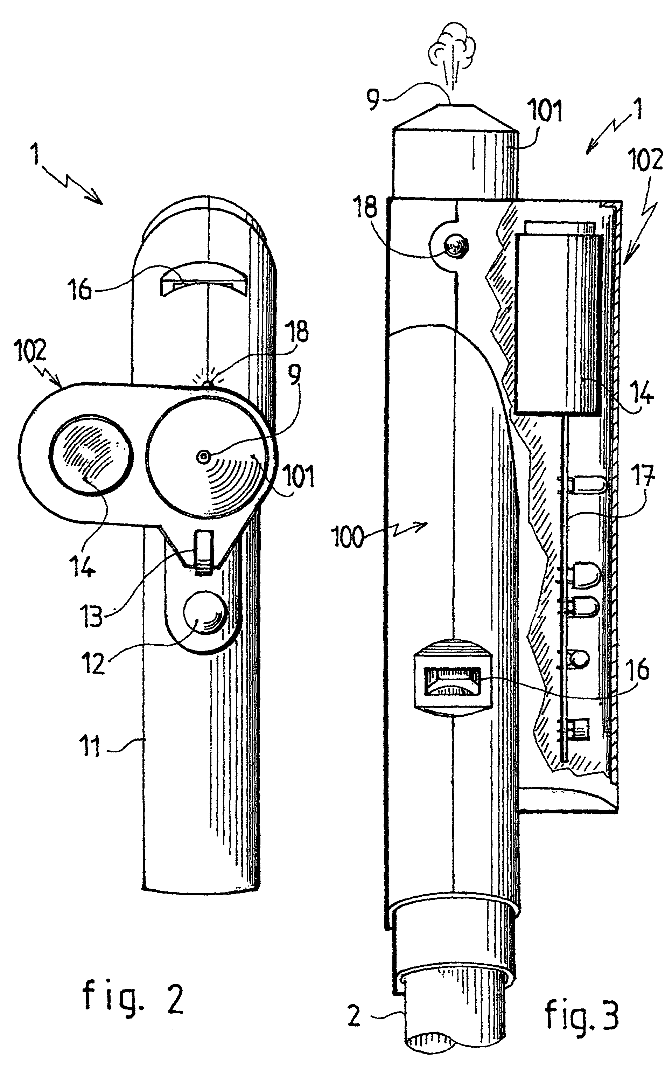

Alignée sous le canon 101 du pistolet 1, une tige 13 servant de guide d'approche, peut coulisser horizontalement depuis une position de repos (représentée sur la figure 1 en traits pleins), à l'intérieur du canon 101 jusqu'à une position de travail (dont la partie arrière, interne au pistolet, est représentée en traits mixtes fins sur la figure 1) correspondant à la distance convenable pour une projection optimale de la carboglace sur la zone à traiter mais encore pour un contrôle thermique juste et précis de ladite zone au moyen d'un pyromètre à infrarouge 14 (représenté sur les figures 2 et 3) comme il sera dit plus loin.

Accessoirement, un interrupteur à lame 15 est prévu à l'intérieur du canon du pistolet 1 pour être normalement ouvert tant que la tige 13 n'est pas entièrement tirée vers l'extérieur, c'est-à-dire en position de travail pour le pistolet ; dès que la tige 13 est totalement tirée, l'interrupteur 15 se trouve en position fermée grâce à un bossage 132 aménagé à l'extrémité interne de la tige 13 mettant alors sous tension le pyromètre à infrarouge d'une part, et autorisant le déclenchement de l'électrovanne 7 par action de l'interrupteur de commande 12.

Ainsi constitué, le pistolet cryogénique 1 utilisable notamment en cryothérapie appliquée à l'homme ou à l'animal d'ailleurs, est particulièrement indiqué dans les urgences sportives pour traiter la douleur, les contusions, les entorses, les oedèmes, les hématomes, les claquages musculaires, les crampes, les contractures, les luxations acromio-claviculaires, ou encore dans les soins aigus pour traiter les inflammations réactionnelles, les tendinites, les bursites, et autres ténosynovites, périostites, etc ...

Dans tous ces cas d'utilisation de l'appareil autonome et portable conforme à l'invention, il suffit d'introduire à l'arrière du pistolet 1 une cartouche de CO₂ liquéfié sous pression, préparé conformément aux normes et prescriptions pour une utilisation médicalisée en s'assurant que la cartouche est bien positionnée sur son support 3, grâce à une fenêtre de visualisation 16 prévue par exemple sur la partie supérieure du pistolet 1. L'opérateur sort ensuite la tige 13 servant de guide d'approche, en la tirant vers l'extérieur dans le prolongement du canon jusqu'à une butée 131, procurant ainsi un organe pratique pour maintenir l'appareil à bonne distance de la zone à traiter ; en outre, la butée 131 forme un bossage 132 capable de repousser une lamelle 151 commandant l'interrupteur général 15 pour mettre sous tension l'électronique 17 (figure 3) qui gouverne les principales fonctions de l'appareil 1. Ledit appareil pris en main par l'utilisateur grâce à sa crosse ergonomique 11 est normalement tenu de telle manière que le canon 101 du pistolet 1 soit sensiblement horizontal, ce qui a pour effet automatique de maintenir constamment la cartouche 2 tête en bas, avec une inclinaison verticale α d'au moins 15° par rapport au plan horizontal assurant au moment de l'utilisation, de ne soutirer de la cartouche 2 que du CO₂ en phase liquide et en aucun cas, en phase vapeur. Pendant l'utilisation, il suffit d'incliner le canon du pistolet 1 vers le bas en direction de la zone douloureuse ou de l'inflammation à traiter (ce qui a pour conséquence heureuse d'aggraver encore l'inclinaison α de la cartouche), en maintenant l'extrémité 9 de sortie du tube de détente et d'éjection 8 à une distance telle que l'extrémité distale 133 de la tige 13 soit pratiquement au contact de la zone à traiter. L'opérateur actionne ensuite l'interrupteur 12 à la manière d'une gâchette pour commander l'électrovanne 7 et soutirer le CO₂ liquide de la cartouche 2 pour l'amener au travers du tube du détente 8 vers l'extrémité 9 et le projeter, sur la zone de traitement, sous forme de carboglace, par un balayage continu et croisé au-dessus de la zone à traiter ; la carboglace sur la peau du patient est alors sublimée et créé un abaissement de température très fort par enlèvement de calories lors de l'opération de sublimation comme il a été dit. Lorsque le pyromètre à infrarouge 14, fonctionnant comme il sera dit plus loin, détecte que la température de la zone traitée approche les 2°C, il donne une consigne à l'électronique 17 qui alerte l'utilisateur d'un danger imminent de brûlure grâce à un dispositif d'alarme sonore et/ou lumineuse comme par exemple une LED 18 (figure 1, 2 et 3) ; lorsque la LED 18 clignote, il suffit d'écarter légèrement le pistolet de manière à éviter dans tous les cas de passer en dessous de 0°C au niveau de la peau du patient ; généralement la durée de traitement se situe autour de 30 à 50 secondes.

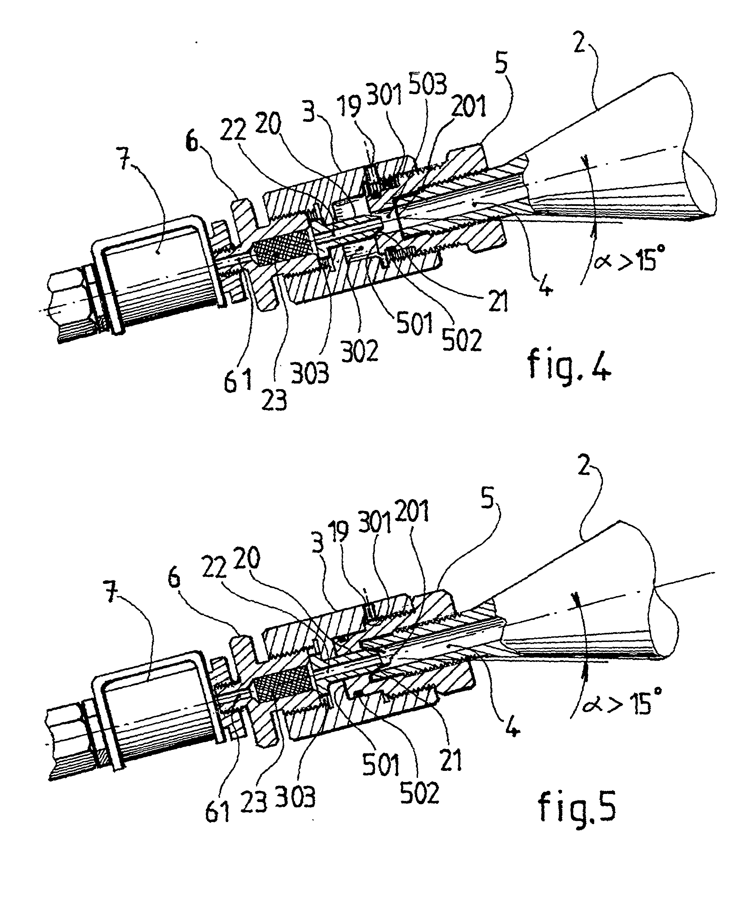

Lorsque la cartouche 2 est épuisée, c'est-à-dire qu'elle ne contient plus de CO₂ en phase liquide, il est nécessaire de la remplacer ou de la recharger ; on sait qu'à ce moment là, la cartouche 2 contient encore de l'anhydride carbonique sous forme gazeuse qui n'a, comme on l'a dit, plus aucun intérêt en cryothérapie ; il est donc important de purger totalement le gaz résiduel à l'intérieur de la cartouche 2 avant son total retrait de son support 3 qui comporte à cet effet un trou de purge 19 (figure 4) assurant la purge du gaz résiduel dans la cartouche 2 pendant son dévissage et ce, avant sa complète désolidarisation du pistolet 1.

Il va de soi que l'alarme visuelle 18 commandée par le pyromètre à infrarouge 14 pourrait être remplacée ou complétée par une alarme sonore de toute manière que l'homme de l'art saurait adapter sans difficulté ; de même, il est évident que pour des utilisations tout à fait ponctuelles, il peut être possible d'utiliser la consigne du pyromètre à infrarouge 14, lorsqu'il a détecté que la zone de traitement a atteint 2°C, pour commander automatiquement l'alimentation électrique de l'appareil bloquant ainsi la projection de carboglace ; toutes autres adaptations du même type entreraient naturellement dans le cadre de l'invention.

On décrira maintenant plus en détail la chaîne de détente du CO₂ liquide à l'intérieur du pistolet 1 et ce, en référence notamment aux figures 1, 4 et 5.

La cartouche de CO₂ médicalisé le cas échéant et liquéfié sous pression, est en fait une cartouche comportant classiquement un récipient métallique de forme cylindrique tout à fait analogue à ce que l'on rencontre dans de nombreux équipements comme les extincteurs, muni d'une tête de soutirage, agencée pour coopérer avec un support d'utilisation. Cet e cartouche peut être jetable ou encore rechargeable c'est-à-dire vendue et rechargée ultérieurement par des gaziers de manière tout à fait connue.

Selon une exécution préférée du pistolet conforme à l'invention, celui-ci sera équipé de cartouches jetables contenant 160 g de CO₂ correspondant à un traitement de 40 à 50 secondes et dispensant les utilisateurs d'avoir recours à une logistique lourde pour recharger les bouteilles vides. Cette exécution particulière a conduit à concevoir un système original de soutirage comprenant un système de percussion automatique de la cartouche pour limiter des manipulations hasardeuses, et donc à modifier l'écrou standard des cartouches du commerce, vendues par exemple par la société française VALLOUREC, afin d'assurer l'étanchéité pendant et après la perforation de l'opercule en bronze 201 obturant la cartouche jetable 2, comme il sera dit plus loin. On peut remarquer ici que le pas de vis de l'écrou 5 venant se visser au-dessus de la tête de soutirage 4 de la cartouche 2, a été volontairement choisi parmi des pas peu courant de manière à ce qu'il ne soit pas possible d'utiliser des cartouches non adaptées au pistolet 1.

Le système de percussion des cartouches 2. se décompose en trois parties conformément aux figures 4 et 5. Un support cylindrique 3 comportant du côté cartouche une première chambre d'entrée 301 filetée intérieurement, dans laquelle vient se visser l'écrou 5 préalablement monté sur la tête de soutirage 4 de la cartouche 2 qui est obturée par un opercule en bronze 201 ; dans l'axe de cette première chambre 301 et y débouchant, une deuxième chambre 302 de diamètre plus petit vient collaborer avec l'extrémité 501 de l'écrou 5 de diamètre plus faible que la partie filetée dudit écrou afin d'assurer une étanchéité totale, grâce à un joint torique extérieur 502 monté sur ladite extrémité 501 entre la cartouche 2 et le reste du dispositif dès que le percuteur 20 perfore l'opercule en bronze 201. Le percuteur 20 formant la deuxième partie du système, est constitué d'une pointe 21 fixe et solidaire du support 3 qui peut s'engager dans la partie d'extrémité creuse 503 de l'écrou 5 entourant la tête de soutirage 4 jusqu'à venir au contact de l'opercule 201 lorsque l'engagement dudit écrou 5 dans le support 3 est suffisant pour assurer la solidarisation de l'ensemble ; à cet instant, la pointe 21 est en contact avec l'opercule 201 et le joint torique 202 est au contact des parois de la chambre 302 assurant son étanchéité avec la tête de soutirage 4 ; il suffit alors, comme représenté sur la figure 5, de continuer à visser l'ensemble cartouche 2/écrou 5 à l'intérieur du support 3 pour perforer l'opercule 201, le CO₂ pouvant alors se détendre vers la partie aval du support 3 en passant d'abord par un canal longitudinal 22 prévu à cet effet dans l'axe du percuteur 20 qui débouche dans une troisième chambre coaxiale 303 du support 3 nécessaire à la mise en place du système de percussion 20 et supportant dans l'écoulement du CO₂ liquide, un filtre 23 destiné à retenir d'éventuels résidus d'usinage provenant par exemple de la cartouche 2. Le filtre 23 est coiffé par un écrou 6 coopérant avec un filetage interne de la chambre 303 de manière classique. L'écrou 6 traversé axialement par un canal 61 est muni sur sa face aval d'un axe fileté permettant d'implanter à la suite l'entrée de l'électrovanne 7.

Cette dernière, choisie parmi des électrovannes haute-pression, permet l'ouverture et la fermeture d'un canal axial prolongeant le précédent 61 par simple commande électrique d'un électro-aimant disposé dans l'axe de l'électrovanne. A la sortie de l'électrovanne 7 on monte suivant un assemblage bien connu un tube de détente 8 légèrement courbé pour compenser l'inclinaison α de la cartouche sur son support 3, reliant ainsi la tête de soutirage 4 désoperculée au point d'éjection 9 du pistolet 1. On notera que jusqu'à la sortie de l'électrovanne 7, le CO₂ est toujours sous sa forme liquide et il n'y a pas formation de glaçon susceptible d'empêcher la circulation du fluide ; dans le but de réguler le débit de sortie du CO₂ liquide, on donne au tube de détente 8 un diamètre intérieur très faible, de l'ordre de 0,5 mm. Afin d'éviter la formation d'un bouchon de glace à la sortie du détendeur, on utilise préférentiellement un tube en PTFE.

Compte tenu de la pression du CO₂ à la sortie de la cartouche 2 qui est de l'ordre de 50 bars, la vitesse d'écoulement du CO₂ à l'intérieur du tube de détente 8 est suffisamment grande pour évacuer à l'extérieur la carboglace en particules micronisées se sublimant dans la zone d'utilisation ou la zone de soin.

Lorsque la cartouche 2 a délivré l'intégralité de son contenu en phase liquide, il est nécessaire de procéder à son remplacement ou, selon une autre exécution, à son remplissage. Pour ce faire, il convient de retirer la cartouche 2 de son support 3 et le problème s'est posé d'évacuer préalablement le gaz résiduel à l'intérieur de la cartouche avant son retrait complet, afin d'éviter un recul brusque pouvant être en outre accompagné d'une détonation. Pour purger la cartouche avant son retrait, il a donc été prévu un trou de purge 19 reliant l'extérieur et la chambre 301 disposée la plus en amont du support 3 (c'est-à-dire la chambre ne comprenant pas l'organe de percussion 20) ; ce trou de purge 19 (figure 4) assure la purge du gaz résiduel au moment où l'on retire la cartouche 2 en dévissant l'écrou 5 de son logement dans la chambre 301 du support 3 ; naturellement, la géométrie intérieure de cette chambre 301 a été prévue pour que, dès que la tête désoperculée de la cartouche 2 se dégage de la pointe de percussion 21, le gaz résiduel soit en contact avec le trou de purge 19, c'est-à-dire avec l'extérieur, alors même que la longueur du filetage de l'écrou 5 encore engagée dans la chambre 301 est encore suffisante pour maintenir rigidement l'ensemble et éviter tout recul brutal de la cartouche libérée de son support ; il suffira donc dès que le sifflement du gaz au travers du trou de purge 19 apparaît, d'attendre qu'il s'arrête pour continuer à dévisser l'écrou 5 et retirer la cartouche 2 en vue de son remplacement.

Selon une caractéristique particulièrement importante de l'invention, le pistolet 1 qui vient d'être décrit est en outre complété par un organe de contrôle de la température de la zone d'utilisation afin d'éviter que l'abaissement de température favorable pour le traitement n'occasionne des dégâts tissulaires ; à cet effet et selon une exécution préférée de l'invention, il est prévu sur le pistolet parallèlement à son canon 101 incorporant la chaîne de détente du CO₂ un second canon 102 recevant un détecteur de seuil de température 14 et son électronique de commande 17 apte à fonctionner à distance sans contact avec la zone d'utilisation ou de soin, conformément aux figures 2 et 3 des dessins. Selon une exécution préférée, dans l'exemple particulier d'un pistolet destiné à la cryothérapie, on choisit comme détecteur de seuil de température associé au pistolet, un pyromètre à infrarouge 14 fonctionnant de préférence avec une alimentation continue de 12 volts comme l'électrovanne 7 ; l'optique du pyromètre 14 est disposée de telle manière que le champ de vision corresponde aussi complètement que possible à la cible à mesurer sur la zone d'utilisation ou sur la peau afin d'obtenir une lecture de température juste et précise. A cet effet, l'optique du pyromètre 14 est calculée pour qu'à un diamètre de cible de 15 mm sur la zone de traitement corresponde un spot de focalisation sur le détecteur ayant un diamètre de 1 mm environ, et ce, compte tenu d'une distance entre la lentille du pyromètre et la cible comprise entre 120 et 150 mm correspondant, comme on l'a vu, à une position optimale de l'appareil par rapport à la zone d'utilisation ; en effet, si la distance de traitement est trop courte, le gradient thermique de la peau devient tellement important que la température de la zone traitée et la température de la zone scrutée par le pyromètre sont très éloignée, avec la conséquence de rendre aléatoire la consigne d'alarme. D'un autre côté, un éloignement trop grand aboutirait à peu près sûrement à une superposition entre le jet de carboglace et le rayonnement infrarouge, avec la conséquence que le pyromètre relèverait plus sûrement la température du jet que celle de la zone traitée. A cet égard, il est, en outre, prévu de décaler les deux canons 101 et 102, le canon 102 qui contient le pyromètre 14 étant en retrait par rapport à l'autre, pour éviter toute contamination de l'optique par la projection du CO₂.

Le détecteur de seuil du pyromètre 14 est réglé à la température fixe de +2 °C afin de conserver une marge de sécurité par rapport à la température critique de 0°C comme il a déjà été dit. Pour se faire, le pyromètre 14 est couplé à une électronique 17 (figure 3) fonctionnant aussi sous une alimentation de 12 volts par exemple dès que le guide d'approche 13 est complètement tiré à l'extérieur du pistolet ; ainsi il est possible de régler l'émissivité du détecteur se définissant comme le rapport de l'énergie rayonnée par la zone de traitement dont on mesure la température et de l'énergie émise par un corps noir à la même température. S'agissant par exemple de la peau qui est une matière organique, on sait que son émissivité est de 0,95, de sorte que le pyromètre 14 sera pré-réglé à cette valeur.

On comprend de la même manière que le pyromètre doive répondre assez vite pour effectuer le contrôle de la température ; pour autant, le temps de réponse du pyromètre ne doit pas être trop court afin d'écrêter les valeurs extrêmes de températures à l'occasion du nécessaire balayage de la surface traitée comme il a été dit plus haut. Dans ces conditions, le temps de réponse sera pré-réglé à 5 secondes environ.

Lorsque le seuil de +2°C est atteint, c'est-à-dire dès que la température de la zone à traiter passe en dessous de la valeur de 2°C, l'électronique 17 déclenche une alarme sonore et/ou visuelle telle qu'une LED 18 afin de prévenir l'utilisateur soit d'arrêter la pulvérisation, soit d'écarter momentanément le pistolet 1 de la zone de traitement. Naturellement on comprend bien que toutes les variantes d'exécution pourraient être envisagées, comme par exemple le blocage automatique de la pulvérisation en coupant l'alimentation de l'électrovanne 7 dès que le seuil de température est atteint.

Il va de soi que les caractéristiques du pyromètre à infrarouge 14 qui viennent d'être données ne constituent qu'un exemple préféré de mise en oeuvre de l'invention et que tous autres réglages répondant à des besoins particuliers ou ponctuels pourraient être envisagés sans sortir du cadre de l'invention ; de même, il pourrait être utilisé d'autres détecteurs de température à distance sans changer les caractéristiques essentielles de l'invention. Enfin, on pourrait envisager de remplacer l'anhydride carbonique sous pression, par d'autres gaz liquéfiés comme par exemple l'argon ; à ce jour, et bien que théoriquement on puisse obtenir des résultats sensiblement identiques, l'anhydride carbonique, reste et de loin, le gaz le mieux adapté pour l'appareil portable et autonome de cryothérapie, qu'il soit utilisé dans les soins destinés à l'homme ou à l'animal, ou d'autres applications cryogéniques en dehors du milieu médical.

## Revendications

1. Appareil autonome (1) de cryogénie utilisant à titre principal la température de détente, de l'anhydride carbonique (CO₂) en phase liquide/solide ou équivalent, comportant un réservoir (2) de CO₂ liquéfié sous pression dont la tête de soutirage (4) est reliée à un système de détente et d'éjection du CO₂ liquide/solide de telle manière que pendant toute la durée d'utilisation, seule la partie liquide du CO₂ à l'intérieur du réservoir (2) vienne au contact de ladite tête (4) pour y être soutirée et propulsée vers la zone d'utilisation et comportant des organes de commande de détente et de contrôle de la température de la zone d'utilisation **caractérisé en ce que** le réservoir ayant la forme générale d'une cartouche (2) de gaz comprimé munie à son extrémité de la tête de soutirage (4) comprenant des moyens (3,5) de solidarisation et de désolidarisation avec l'appareil (1) et des organes (6,7) de mise en communication avec son système de détente et d'éjection (8), est situé au-dessus de l'appareil (1), lorsqu'il est en position sensiblement horizontale, c'est-à-dire qu'il est pris en main pour une intervention, l'axe de la cartouche (2) se trouvant alors incliné dans le plan vertical et la tête de soutirage (4) étant tournée vers le bas en direction de l'appareil auquel elle est solidarisée.

2. Appareil selon la revendication 1 **caractérisé en ce que** des moyens sont agencés pour que l'inclinaison verticale de l'axe de la cartouche par rapport au plan horizontal soit d'au moins 15° pendant l'intervention.

3. Appareil selon l'une quelconque des revendications 1 ou 2 **caractérisé en ce que** la tête de soutirage (4) de la cartouche (2), qui est constituée d'un embout cylindrique fileté extérieurement et fermé par un opercule (201) par exemple en bronze, est solidarisée au support de cartouche (3) dans l'appareil par l'intermédiaire d'une pièce (5) assurant le montage lorsque la cartouche (2) est vissée dans le support (3) pour amener d'abord l'opercule (201) au contact d'un organe (21) de percussion (20) avantageusement fixe, s'étendant dans l'axe du support (3), puis pour percer ledit opercule en assurant l'étanchéité du montage, afin de soutirer le CO₂ en phase liquide par un canal axial (22) traversant de part en part le dispositif de percussion (20), relié à son extrémité libre au système de détente (8), un trou de purge (19) entre l'espace intérieur du support (3) et l'extérieur étant prévu radialement dans la partie (301) dudit support ne comprenant pas l'organe de percussion (21) pour purger le gaz CO₂ résiduel dans la cartouche vide pendant son dévissage et avant sa complète désolidarisation de l'appareil.

4. Appareil selon l'une quelconque des revendications 1, 2 ou 3 **caractérisé en ce que** l'organe de mise en communication entre la cartouche (2) et le système de détente (8) est une électrovanne (7), de préférence en ligne, fonctionnant en "tout ou rien".

5. Appareil selon l'une quelconque des revendications précédentes, **caractérisé en ce que** le système de détente du CO₂ liquide ou équivalent et d'éjection (9) du CO₂ solide ou équivalent est constitué d'une tubulure (8) de faible diamètre intérieur, de l'ordre de 0,5 mm, dans laquelle la vitesse d'écoulement du CO₂ ou équivalent est suffisamment grande pour évacuer à l'extérieur le CO₂ solide ou équivalent en particules micronisées se sublimant dans la zone d'utilisation.

6. Appareil selon l'une quelconque des revendications précédentes, **caractérisé par** sa forme générale de pistolet (1) comprenant au moins une coque recevant à l'arrière la cartouche (2), un canon (101) et une crosse (11) munie d'une gâchette (12) de commande générale dont l'ergonomie est telle que le pistolet (1) étant normalement tenu en main par l'utilisateur, le canon (101) soit sensiblement horizontal, maintenant automatiquement la cartouche (2) inclinée d'un angle α tête vers le bas, cette inclinaison ne pouvant que s'accentuer en utilisation.

7. Appareil selon la revendication 6 **caractérisé en ce que** à l'intérieur du canon (101) on loge le support (3) de cartouche débouchant sur l'arrière et l'ensemble des organes (4,5,6,7,8) de la chaîne de détente aboutissant à l'avant (9), au système d'éjection du CO₂ liquide ou équivalent, **en ce que** à l'intérieur de la crosse, on loge les organes d'alimentation de contrôle à savoir les batteries d'alimentation (10) et un interrupteur de commande (12) d'éjection en lieu et place de la gâchette, et **en ce qu'**on attache parallèlement au canon (101) un détecteur (14) de température de la zone d'utilisation, avantageusement et justement décalé en arrière du point d'éjection (9) pour éviter toute interaction entre le fonctionnement du détecteur (14) et le flux de CO₂ propulsé ou équivalent.

8. Appareil selon l'une quelconque des revendications précédentes, **caractérisé en ce que** l'organe de contrôle de la température de la zone d'utilisation est sans contact avec celle-ci.

9. Appareil selon la revendication précédente **caractérisé en ce que** le détecteur de l'organe de contrôle de la température est un pyromètre à infrarouge (14).

10. Appareil selon la revendication 9 **caractérisé en ce que** la distance focale du pyromètre à infrarouge (14) est telle d'une part qu'il n'y ait pas d'interaction avec la pulvérisation de la carboglace, et d'autre part que la mesure précise de température se fasse lorsque l'appareil (1) est positionné à distance convenable pour l'utilisation envisagée.

11. Appareil selon la revendication 10 **caractérisé en ce que** la distance convenable entre l'appareil (1) et la zone d'utilisation est donnée par un guide d'approche.

12. Appareil selon la revendication 11 **caractérisé en ce que** le guide d'approche est constitué d'une tige (13) alignée sous le canon (101) du pistolet (1) et coulissante depuis une position de repos à l'intérieur du canon jusqu'à une position de travail externe au canon correspondant à une distance convenable pour une projection optimale du CO₂ en phase liquide/solide ou équivalent.

13. Appareil selon la revendication 12 **caractérisé en ce que** la tige (13) qui est à cet effet, munie d'au moins un bossage (132) servant de butée de sortie (131) à son déplacement, coopère avec un interrupteur électrique (15) dont la fermeture est obtenue par ledit bossage (132) pour autoriser la mise sous tension de l'appareil.

14. Appareil selon l'une quelconque des revendications 9 à 13 **caractérisé en ce que** le pyromètre à infrarouge est couplé à une alarme visuelle (18) et/ou sonore lorsque la baisse de température de la zone d'utilisation atteint le seuil de 2°C, le pyromètre pouvant alors avantageusement, mais pas nécessairement, couper automatiquement l'alimentation électrique de l'appareil.

15. Appareil selon l'une quelconque des revendications 9 à 14 **caractérisé en ce que** pour un diamètre de cible égal à environ 15 mm sur la zone d'utilisation, on pré-règle le pyromètre (14) à un taux d'émissivité de 0,95, à un temps de réponse de 5 secondes et on choisit une optique pour avoir une distance focale comprise entre 120 et 150 mm.

## Claims

1. Self-driven cryogenic appliance principally using the expansion temperature of carbon anhydride (CO₂) in liquid/solid phase or equivalent, comprising a reservoir (2) of liquefied CO₂ under pressure whose extraction head (4) is connected to a system for expansion and ejection of liquid/solid CO₂ so that during its entire duration of use solely the liquid part of the CO₂ inside the reservoir (2) comes into contact with said head (4) to be extracted therefrom and propelled towards the zone of use, and comprising members for expansion command and zone of use temperature control, **characterized in that** the reservoir, having the general shape of a compressed gas cartridge (2) provided at its end with extraction head (4) and comprising means (3,5) for its securing to and separation from appliance (1) and members (6,7) enabling communication with its expansion and ejection system (8), is located above the appliance when it is in substantially horizontal position, i.e. it is hand held for use, the axis of cartridge (2) then being inclined in the vertical plane and the extraction head (4) facing downwards in direction of the appliance to which it is secured.

2. Appliance as in claim 1, **characterized in that** means are arranged so that the vertical inclination of the cartridge axis relative to the horizontal plane is at least 15° when in use.

3. Appliance as in either of claims 1 or 2,
**characterized in that** the extraction head (4) of cartridge (2) which consists of a cylindrical nozzle with outer thread and closed by a cap (201) in bronze for example, is secured to the cartridge holder (3) in the appliance via a part (5) ensuring assembly when cartridge (2) is screwed into holder (3) to bring cap (201) firstly into contact with a percussion member (21) advantageously fixed and extending into the axis of holder (3), then to pierce said cap ensuring the imperviousness of the assembly so as to extract CO₂ in liquid phase via an axial channel (22) crossing through the percussion device (20), connected at its free end to the expansion system (8), a purge hole (19) between the inner space of holder (3) and the outside being provided radial fashion in part (301) of said holder not comprising percussion member (21) to purge residual CO₂ gas from the empty cartridge when it is being unscrewed and before it is completely separated from the appliance.

4. Appliance as in any of claims 1, 2 or 3,
**characterized in that** the member setting up communication between cartridge (2) and the expansion system (8) is an electronic valve (7), preferably on line with input/output operation.

5. Appliance as in any of the preceding claims, **characterized in that** the system for expansion of liquid CO₂ or equivalent and ejection of solid CO₂ or equivalent (9) consists of tubing (8) of narrow inner diameter in the order of 0.5 mm in which the flow rate of CO₂ or equivalent is sufficiently high to evacuate the solid CO₂ or equivalent towards the outside in micronized particles which sublimate in the zone of use.

6. Appliance as in any of the preceding claims, **characterized by** its general shape of a gun (1) comprising at least one body receiving cartridge (2) at the rear, a barrel (101) and a grip (11) provided with a general command trigger (12) designed such that the gun (1) normally being hand-held by the user, barrel (101) is therefore substantially horizontal and automatically maintains cartridge (2) inclined head downwards by an angle □, this inclination only able to accentuate during use.

7. Appliance as in claim 6, **characterized in that** on the inside of barrel (101) the cartridge holder (3) is housed leading to the rear and the set of members (4,5,6,7,8) of the expansion chain leading to the front (9) towards the ejection system of liquid CO₂ or equivalent, **in that** inside the grip the command power supply members are housed, namely the power batteries (10) and en ejection command switch (12) in lieu and stead of a trigger, and **in that** parallel to barrel (101) a temperature detector (14) is attached detecting temperature of the zone of use, advantageously just set back from the ejection point (9) to avoid any interaction between functioning of the detector (14) and the flow of propelled CO₂ or equivalent.

8. Appliance as in any of the preceding claims, **characterized in that** the member controlling the temperature of the zone of use is not in contact with this zone.

9. Appliance as in the preceding claim, **characterized in that** the detector of the temperature controlling member is an infrared pyrometer (14).

10. Appliance as in claim 9, **characterized in that** the focal distance of the infrared pyrometer (14) is such firstly that there is no interaction with the spraying of carbon ice, and secondly that precise temperature measurement is made when the appliance (1) is located at a suitable distance for intended use.

11. Appliance as in claim 10, **characterized in that** the suitable distance between the appliance (1) and the zone of use is given by an approach guide.

12. Appliance as in claim 11, **characterized in that** the approach guide consists of a rod (13) aligned underneath the barrel (101) of gun (1) and slides from a rest position inside the barrel to a work position outside the barrel corresponding to a suitable distance for optimal spraying of CO₂ in liquid/solid phase or equivalent.

13. Appliance as in claim 12 **characterized in that** the rod (13), which for this purpose is provided with at least one boss (132) acting as limit stop (131) for its movement, cooperates with an electric switch (15) which is closed by said boss (132) to authorize powering up of the appliance.

14. Appliance as in any of claims 9 to 13, **characterized in that** the infrared pyrometer is coupled to a visual and/or sound alarm (18) when the drop in temperature of the zone of use reaches the threshold of 2°C, the pyrometer then advantageously but not necessarily possibly cutting out the electric power supply to the appliance automatically.

15. Appliance as in any of claims 9 to 14, **characterized in that** for a target diameter of approximately 15 mm on the zone of use, the pyrometer (14) is pre-set to an emissivity of 0.95, a response time of 5 seconds and the optics are chosen to obtain a focal distance of between 120 and 150 mm.

## Patentansprüche

1. Autonomes Kryo-Gerät (1) mit hauptsächlicher Verwendung der Entspannungstemperatur von Kohlendioxid (CO₂) in flüssiger/fester Phase oder ähnlichem, das einen Behälter (2) für verflüssigtes CO₂ unter Druck umfasst, dessen Entnahmekopf (4) mit einem System zur Entspannung und zum Ausstoß von flüssigem/festem CO₂ so verbunden ist, dass während der gesamten Nutzungsdauer nur der flüssige Teil des CO₂ im Innern des Behälters (2) mit dem besagten Kopf (4) in Kontakt tritt, um dort entnommen und in Richtung des Anwendungsbereichs getrieben zu werden, und Steuereinrichtungen zur Entspannung und Überprüfung der Temperatur im Anwendungsbereich, **dadurch gekennzeichnet, dass** sich der allgemein als Patrone (2) geformte Behälter für komprimiertes Gas, der an seinem Ende mit dem Mittel (3, 5) zur Verbindung und Lösen der Verbindung mit dem Gerät (1) und Organen (6, 7) zur Herstellung der Kommunikation mit seinem Entspannungs- und Ausstoßsystem (8) aufweisenden Entnahmekopf (4) versehen ist, oberhalb des Geräts (1) befindet, wenn sich dieses in etwa waagerechter Position befindet, d. h. für einen Eingriff in die Hand genommen wird, wobei die Achse der Patrone (2) dann senkrecht geneigt ist und der Entnahmekopf (4) nach unten in Richtung des Geräts, mit dem er verbunden ist, gerichtet wird.

2. Gerät nach Anspruch 1, **dadurch gekennzeichnet, dass** die Mittel angeordnet sind, damit die senkrechte Neigung der Achse der Patrone in bezug auf die waagerechte Ebene während des Eingriffs mindestens 15 Grad beträgt.

3. Gerät nach einem der Ansprüche 1 oder 2, **dadurch gekennzeichnet, dass** der Entnahmekopf (4) der Patrone (2), der von einem zylindrischen, außen mit einem Gewinde versehenen Endstück gebildet wird, das von einem zum Beispiel aus Bronze bestehenden Verschlussorgan (201) verschlossen wird, mit dem Patronenhalter (3) im Gerät mittels eines Teils (5) verbunden wird, das die Montage gewährleistet, wenn die Patrone (2) in den Halter (3) eingeschraubt ist, um zunächst das Verschlussorgan (201) in Kontakt mit einem vorteilhafterweise festen Organ (21) zur Perkussion (20) zu führen, das sich in der Achse des Halters (3) erstreckt, um dann das besagte Verschlussorgan bei Gewährleistung der Dichtigkeit des Aufbaus zu durchstoßen, um das CO₂ in flüssiger Phase durch einen die Perkussionsvorrichtung (20) ganz durchquerenden axialen Kanal (22) zu entnehmen, der an seinem freien Ende mit dem Entspannungssystem (8) verbunden ist, wobei eine Entlüftungsöffnung (19) zwischen dem Innenraum des Halters (3) und außen radial in dem Teil (301) des besagten Halters vorgesehen ist, der kein Perkussionsorgan (21) aufweist, um das restliche CO₂ in der leeren Patrone beim Abschrauben und vor deren vollständiger Trennung vom Gerät abzulassen.

4. Gerät nach einem der Ansprüche 1, 2 oder 3, **dadurch gekennzeichnet, dass** das Kommunikationsorgan zwischen der Patrone (2) und dem Entspannungssystem (8) ein Elektroventil (7) ist, vorzugsweise in Reihe, das nach dem Prinzip "alles oder nichts" funktioniert.

5. Gerät nach einem der vorhergehenden Ansprüche, **dadurch gekennzeichnet, dass** das System zur Entspannung des flüssigen CO₂ oder ähnlichem und zum Ausstoß (9) des festen CO₂ oder ähnlichem von einem Rohrstutzen (8) mit einem kleinen Innendurchmesser in der Größenordnung von 0,5 mm gebildet wird, in dem die Strömungsgeschwindigkeit des CO₂ oder ähnlichem ausreichend groß ist, um das feste CO₂ oder ähnliches in mikronisierten Teilchen nach außen zu befördern, die sich im Anwendungsbereich sublimieren.

6. Gerät nach einem der vorhergehenden Ansprüche, durch seine allgemeine Pistolenform (1) gekennzeichnet, umfassend mindestens eine Schale, die die Patrone (2) hinten aufnimmt, einen Lauf (101) und einen Kolben (11) mit einem Abzug (12) zur allgemeinen Steuerung, dessen Ergonomie dergestalt ist, dass, wenn die Pistole (1) vom Anwender normal in der Hand gehalten wird, der Lauf (101) etwa waagerecht ist und die Patrone (2) automatisch in einem Winkel α mit dem Kopf nach unten gehalten wird, wobei sich diese Neigung bei der Anwendung weiter akzentuieren kann.

7. Gerät nach Anspruch 6, **dadurch gekennzeichnet, dass** man im Lauf (101) den nach hinten überstehenden Patronenhalter (3) platziert und alle Organe (4, 5, 6, 7, 8) der vorn (9) im System zum Ausstoß des flüssigen CO₂ oder ähnlichem endenden Entspannungskette, dadurch, dass man im Kolben die Organe zur Versorgung und Steuerung platziert, nämlich die Versorgungsbatterien (10) und anstelle des Abzugs einen Schalter (12) zum Ausstoß, und dadurch, dass man parallel zum Lauf (101) einen Fühler (14) zum Fühlen der Temperatur im Anwendungsbereich anbringt, der vorzugsweise genau hinter dem Ausstoßpunkt (9) liegt, um jedwede Beeinflussung zwischen der Funktionsweise des Fühlers (14) und dem angetriebenen CO₂-Strom oder ähnlichem zu vermeiden.

8. Gerät nach einem der vorhergehenden Ansprüche, **dadurch gekennzeichnet, dass** das Organ zur Kontrolle der Temperatur im Anwendungsbereich kontaktlos zu dieser ist.

9. Gerät nach dem vorhergehenden Anspruch, **dadurch gekennzeichnet, dass** der Fühler des Temperaturkontrollorgans ein Infrarot-Pyrometer (14) ist.

10. Gerät nach Anspruch 9, **dadurch gekennzeichnet, dass** die Fokaldistanz des Infrarot-Pyrometers (14) einerseits so ist, dass es keine Wechselwirkung mit der Zerstäubung des Trockeneises gibt, und andererseits, dass die präzise Temperaturmessung erfolgt, wenn das Gerät (1) in einem für die beabsichtigte Anwendung angemessenen Abstand positioniert ist.

11. Gerät nach Anspruch 10, **dadurch gekennzeichnet, dass** der angemessene Abstand zwischen dem Gerät (1) und dem Anwendungsbereich von einer Näherungsführung vorgegeben wird.

12. Gerät nach Anspruch 11, **dadurch gekennzeichnet, dass** die Näherungsführung von einem unter dem Lauf (101) der Pistole (1) angeordneten Stab (13) gebildet wird, der aus einer Ruheposition im Lauf in eine Arbeitsposition außerhalb des Laufs verschoben wird, die einem für eine optimale Projizierung des CO₂ in flüssiger/fester Phase oder ähnlichem angemessenen Abstand entspricht.

13. Gerät nach Anspruch 12, **dadurch gekennzeichnet, dass** der Stab (13), der zu diesem Zweck mindestens mit einem bei seiner Verschiebung als Ausgangsanschlag (131) dienenden Buckel (132) versehen ist, mit einem elektrischen Schalter (15) zusammenwirkt, dessen Verschluss durch den besagten Buckel (132) erzielt wird, damit das Gerät unter Spannung gesetzt werden darf.

14. Gerät nach einem der Ansprüche 9 bis 13, **dadurch gekennzeichnet, dass** das Infrarot-Pyrometer mit einem visuellen und/oder akustischen Alarm (18) gekoppelt ist, wenn der Temperaturabfall im Anwendungsbereich die Schwelle von 2 °C erreicht, wobei das Pyrometer dann vorteilhafter-, aber nicht notwendigerweise die elektrische Versorgung des Geräts automatisch abschalten kann.

15. Gerät nach einem der Ansprüche 9 bis 14, **dadurch gekennzeichnet, dass** man das Pyrometer (14) für einen Zieldurchmesser von gleich ca. 15 mm im Anwendungsbereich auf einen Emissionsgrad von 0,95 und eine Reaktionszeit von 5 Sekunden einstellt und man eine Optik für einen Fokalabstand von inklusive 120 bis 150 mm wählt.
